# EUROPEAN PATENT APPLICATION

(11) **EP 4 160 180 A1**
(43) Date of publication of application: **05.04.2023**
(21) Application number: 21818717.7
(22) Date of filing: 30.05.2021
(51) Int. Cl.: G01N 1/10

(54) **LIQUID COLLECTOR AND METHOD FOR COLLECTING LIQUID**

(30) Priority: 01.06.2020 JP 2020095794
(71) Applicant: Provigate Inc., Tokyo 113-0033 (JP)
(72) Inventor: NISHI Mitsumi, Tokyo 113-0033 (JP); MIYAUCHI Noriko, Tokyo 113-0033 (JP)
(74) Representative: Michalski Hüttermann & Partner Patentanwälte mbB
(86) International application number: PCT/JP2021/020576
(87) International publication number: WO 2021/246347

(57) **Abstract**

An embodiment of the present disclosure provides a liquid collector including: an absorber including an elastic material and capable of absorbing liquid in contact with the absorber; a support that supports the absorber; and a tank that receives the liquid discharged from the absorber when the absorber is compressed.

## Description

### Technical Field

The present disclosure relates to a liquid collector and a method for collecting liquid.

### Background Art

A small amount of liquid may be collected by, for example, suction using a dropper or a syringe or absorption into test paper.

It is desirable to collect liquid efficiently. However, when, for example, a small amount of liquid is collected by using a dropper or the like, it may be difficult to efficiently collect all of the liquid.

### Summary of Invention

Here, it is recognized, for example, without limitation, that efficient collection of liquid is desired.

Some embodiments of the present disclosure provide a liquid collector. In some embodiments, the liquid collector includes an absorber capable of absorbing liquid in contact with the absorber. In some embodiments, the absorber includes an elastic material. In some embodiments, the liquid collector includes a support that supports the absorber. In some embodiments, the liquid collector includes a tank. In some embodiments, the tank receives the liquid discharged from the absorber when the absorber is compressed.

Some embodiments of the present disclosure provide a liquid collector. In some embodiments, the liquid collector includes an absorber capable of absorbing liquid in contact with the absorber. In some embodiments, the absorber includes an elastic material. In some embodiments, the liquid collector includes a support that supports the absorber. In some embodiments, the liquid collector includes a first cylinder in which a liquid-receiving space that receives the liquid is defined. In some embodiments, the first cylinder supports the absorber at an end of the first cylinder. In some embodiments, the liquid collector includes a second cylinder into which the first cylinder is insertable. In some embodiments, the second cylinder presses the absorber when the first cylinder is inserted into the second cylinder. In some embodiments, the second cylinder is configured to move the liquid absorbed in the absorber into the liquid-receiving space.

Some embodiments of the present disclosure provide a liquid collector. In some embodiments, the liquid collector includes an absorber capable of absorbing liquid in contact with the absorber. In some embodiments, the absorber includes an elastic material. In some embodiments, the liquid collector includes a first cylinder that supports the absorber at an end of the first cylinder. In some embodiments, the liquid collector includes a second cylinder into which the first cylinder is insertable. In some embodiments, a liquid-receiving space that receives the liquid is defined in the second cylinder. In some embodiments, the second cylinder compresses and deforms the absorber when the first cylinder is inserted into the second cylinder. In some embodiments, the second cylinder is configured to move the liquid absorbed in the absorber into the liquid-receiving space.

Some embodiments of the present disclosure provide a method for collecting liquid. In some embodiments, the method for collecting liquid includes causing an absorber to absorb liquid by bringing the absorber into contact with the liquid. In some embodiments, the method for collecting liquid includes causing the absorber to discharge the liquid absorbed in the absorber. In some embodiments, the method for collecting liquid includes receiving the liquid discharged from the absorber in a tank.

According to the above-described embodiments, for example, the liquid can be efficiently collected.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### Brief Description of Drawings

[Fig. 1] Fig. 1 is a schematic illustration of a liquid collector according to an embodiment.
[Fig. 2] Fig. 2 illustrates an absorber included in the liquid collector illustrated in Fig. 1 in a state such that the absorber is expanded due to absorption of liquid.
[Fig. 3] Fig. 3 illustrates the manner in which the liquid collector illustrated in Fig. 1 is used to collect liquid.
[Fig. 4] Fig. 4 is a flowchart of a method for collecting liquid according to an embodiment.
[Fig. 5] Fig. 5 is a schematic illustration of a liquid collector according to an embodiment.
[Fig. 6] Fig. 6 is a schematic illustration of a liquid collector according to an embodiment.
[Fig. 7] Fig. 7 is a schematic illustration of a liquid collector according to an embodiment.
[Fig. 8] Fig. 8 illustrates an absorber included in the liquid collector illustrated in Fig. 7 in a state such that the absorber is expanded due to absorption of liquid.
[Fig. 9] Fig. 9 illustrates the manner in which the liquid collector illustrated in Fig. 7 is used to collect liquid.
[Fig. 10] Fig. 10 is a schematic illustration of a liquid collector according to an embodiment.
[Fig. 11] Fig. 11 illustrates an absorber included in the liquid collector illustrated in Fig. 10 in a state such that the absorber is expanded due to absorption of liquid.
[Fig. 12] Fig. 12 illustrates the manner in which the liquid collector illustrated in Fig. 10 is used to collect liquid.
[Fig. 13] Fig. 13 is a schematic illustration of a liquid collector according to an embodiment.
[Fig. 14] Fig. 14 is a schematic illustration of a liquid collector according to an embodiment.

### Description of Embodiments

In some embodiments, a liquid collector collects liquid (sometimes also referred to as solution).

In some embodiments, the liquid may be body fluid produced by secretion in a target subject, or liquid other than body fluid. The liquid other than body fluid may be liquid adhering to a target object or liquid not adhering to a target object. The liquid not adhering to a target object may be liquid contained in a target object.

A sample to be collected may be a solution. The liquid may be body fluid, a solution derived from body fluid, or diluted body fluid. The liquid may be a solution other than (not derived from) body fluid, or a mixture of body fluid or a solution derived from body fluid with a solution not derived from body fluid. The solution may be a solution used for sample measurement or a solution used for calibration measurement. For example, the solution may be a standard solution or a calibration solution. The sample to be measured may be a specimen.

Examples of the body fluid include lymph fluid, tissue fluid such as inter-tissue fluid, intercellular fluid, and interstitial fluid, coelomic fluid, serous cavity fluid, pleural fluid, ascites fluid, pericardial fluid, cerebrospinal fluid, synovial fluid, and aqueous humor. Examples of the body fluid further include digestive juice, such as saliva, gastric juice, bile, pancreatic juice, and intestinal juice, sweat, tear, nasal mucus, urine, semen, vaginal fluid, amniotic fluid, and milk. The body fluid may be body fluid of an animal or a human. The "body fluid" may be a solution. The solution may include a physiological buffer, such as phosphate-buffered saline (PBS) or N-tris(hydroxymethyl)methyl-2-aminoethanesulfonic acid (TES) buffer, containing a measurement target substance. The solution is not particularly limited as long as the measurement target substance is contained.

In some embodiments, the target subject may include, or may be, a human. In some embodiments, the target subject may include, or may be, a non-human animal. The non-human animal may include, or may be, a mammal. Examples of non-human animals include, but are not limited to, working animals, livestock animals, pet animals, and wild animals.

In some embodiments, the liquid collector includes an absorber capable of absorbing liquid in contact with the absorber. In some embodiments, the absorber may include, or may be, an elastic material. In some embodiments, when the absorber includes an elastic material, the absorber may be a combination of a portion made of the elastic material and a portion made of another material. In some embodiments, the absorber may be configured to expand when the absorber absorbs the liquid. In some embodiments, the absorber may have mechanical, physical, chemical, and biological characteristics, such as elasticity and surface shape, such that the absorber can be brought into contact with, for example, a skin, a mucous membrane, an eyeball, a tongue, gums, a lip, a mucous membrane in an oral cavity, such as an inner cheek, or a mucous membrane in a nasal cavity, a paranasal sinus, etc., of an animal, such as a human, substantially without causing damage thereto.

The term "elastic material" used in this specification generally refers to a material that is elastically deformable when pressed against a target subject or a target object. The elastic material can be brought into contact with a target subject or a target object substantially without causing damage to a surface thereof in normal use.

The term "absorber material" used in this specification generally refers to a material capable of absorbing liquid. In some embodiments, the absorber material may include, or be composed mainly of, cellulose. In some embodiments, the absorber material may include, or be composed mainly of, a sponge. In some embodiments, the absorber material may include an elastic material.

In some embodiments, the liquid collector includes a support that supports the absorber. In some embodiments, the support may have a shaft shape that extends in a longitudinal direction, or a shape other than a shaft shape.

In some embodiments, the support may include a tube that extends in the longitudinal direction. In some embodiments, the support may have a tubular shape that extends in the longitudinal direction.

In some embodiments, the support may have a circular shape or an elliptical shape in cross-section when viewed in the longitudinal direction. In some embodiments, the support may have, for example, a polygonal shape, such as a triangular shape, a quadrangular shape, a pentagonal shape, a hexagonal shape, or an octagonal shape in cross-section.

In some embodiments, the absorber may be disposed at or near an end of the support or at a location other than the end of the support. In some embodiments, when the absorber is disposed at a location other than the end of the support, the absorber may be disposed on a surface of the support. In some embodiments, the absorber may be disposed at the end of the support such that at least a portion thereof is exposed to the outside, or such that the entirety thereof is exposed to the outside. In some embodiments, the absorber may be such that the absorber does not project outward until the absorber absorbs liquid and expands. In some embodiments, the absorber may be disposed in the support until the absorber absorbs liquid and expands. In some embodiments, the absorber may project outward from the support when the absorber absorbs liquid and expands. When the absorber projects outward from the support, at least a portion of the absorber is exposed to the outside.

The term "exposed" used in this specification means that at least a portion of the absorber is visible when the support is viewed from the outside. In other words, even when the absorber does not project from the support and is, for example, disposed in the support, a portion of the absorber is regarded as being exposed to the outside if that portion of the absorber is visible from the outside when the support is viewed in a certain direction.

In some embodiments, when the support has a shaft shape or a tubular shape, the absorber may be fixed to one end of the support in the longitudinal direction in an expandable manner. In some embodiments, the absorber supported by the support may be configured to expand outward from the support.

In some embodiments, the absorber may be expandable inside the support when the absorber absorbs liquid. In some embodiments, the absorber may be at least partially fixed to the support. In some embodiments, the absorber may be fixed to one end of the tube in the longitudinal direction in an expandable manner in the tube. In some embodiments, the absorber may be expandable in the longitudinal direction inside the tube when the absorber absorbs liquid.

In some embodiments, the absorber may be disposed in, for example, a case or a frame having a partial opening. In such a case, for example, the direction in which the absorber expands can be restricted. For example, when the absorber is disposed in a case or a frame having a tubular shape, the direction in which the absorber expands is restricted to a direction in which the case or the frame opens.

In some embodiments, the absorber may include a plurality of absorbers that are stacked together. In some embodiments, the absorbers may be stacked in a direction in which the absorbers expand when liquid is absorbed therein. In some embodiments, the absorbers may be stacked in a direction crossing the direction in which the absorbers expand when liquid is absorbed therein. In some embodiments, the absorbers may differ from each other in at least one of color, shape, material, and other characteristics. In some embodiments, the absorbers may be connected to each other. In some embodiments, the absorbers may be connected to the support with a line-shaped connecting member. In some embodiments, a plurality of layers of absorbers that differ from each other in at least one of color, shape, material, and other characteristics may be provided. The plurality of layers of the absorbers may be formed integrally with each other by performing, for example, co-injection molding in a molding process. In some embodiments, the absorber may include a material that changes color when the material comes into contact with moisture or absorbs liquid. When the color of a portion of the absorber changes due to absorption of liquid, it can be visually or optically confirmed that an amount of liquid corresponding to the size of a region in which color has changed is absorbed. When the color of the entirety of the absorber changes, it can be visually or optically confirmed that a sufficient amount of liquid is absorbed in the absorber.

In some embodiments, the support including the tube may include a fixing portion (sometimes also referred to as a fixing member) that fixes the absorber in the tube. The fixing portion may be, for example, without limitation, a projection that projects into the absorber in the tube, adhesion between the absorber and the tube, or adhesion between the absorber and a fixing surface that is provided in the tube and extends along a plane crossing the longitudinal direction. In some embodiments, the projection that serves as the fixing portion and projects into the absorber in the tube may project inward from an inner peripheral surface of the tube. In some embodiments, the projection that serves as the fixing portion and projects into the absorber in the tube may include a plurality of projections that are arranged in a circumferential direction of the tube with intervals therebetween, or be formed to extend continuously in the circumferential direction of the tube.

In some embodiments, the support may include a stopper (sometimes also referred to as a fixing portion or a fixing member). In some embodiments, the stopper may determine the position of the absorber with respect to the support. In some embodiments, the stopper may be configured to receive the absorber when the absorber is compressed. In some embodiments, the stopper may have a stopper surface that crosses a direction in which the absorber is compressed. In some embodiments, the stopper may be plate-shaped and extend along a plane crossing the longitudinal direction of the tube of the support. In some embodiments, the stopper may have a flow hole that provides fluid communication between a side adjacent to the absorber and a side opposite to the side adjacent to the absorber in the longitudinal direction of the tube of the support. In some embodiments, the stopper may have one or more flow holes. When the stopper has a plurality of flow holes, a portion or the entirety of the stopper may include, for example, without limitation, a porous body, a mesh, a grid, or a grating-shaped member having a plurality of flow holes. In some embodiments, the stopper may have a two-dimensional structure (flat surface) or a three-dimensional structure in a longitudinal cross-sectional direction of the support.

In some embodiments, the support may have an opening at an end opposite to an end at which the absorber is fixed in the longitudinal direction, and may include a closing body that is removably attachable to the opening or capable of covering and uncovering the opening.

In some embodiments, the liquid collector includes a tank. In some embodiments, the tank receives the liquid discharged from the absorber. In some embodiments, the tank may include a tube that extends in the longitudinal direction, or may have other shapes. In some embodiments, the tank may have a shape of a dish, a bowl, a tray, etc., having a recess capable of receiving the liquid.

In some embodiments, the inside of the tube of the support may serve as the tank. In some embodiments, the tank may be disposed on a side of the stopper having the flow hole, the side being opposite to a side on which the absorber is disposed. In some embodiments, the tank may be a member separate from the support. In some embodiments, the tank may be attachable to the support at an end at which the absorber is disposed, and may be capable of receiving the liquid discharged from the absorber.

In some embodiments, the liquid collector may include a compressing member that compresses and deforms the absorber. The absorber in which the liquid is absorbed may be compressed so that the liquid is discharged therefrom. In some embodiments, when the support includes the tube that extends in the longitudinal direction, the compressing member may be movable back and forth relative to the absorber in the longitudinal direction.

In some embodiments, the compressing member may include a pressing plate that is disposed to face the absorber and that presses the absorber. In some embodiments, the compressing member may include an outer wall having a tubular shape that extends in a direction crossing the pressing plate. In some embodiments, the support may be insertable into a space inside the outer wall with the absorber facing the pressing plate.

In some embodiments, the liquid collector may further include a pressing body that is disposed to face the absorber and that presses the absorber. In some embodiments, the tank may be disposed on a side of the pressing body opposite to a side on which the absorber is disposed. In some embodiments, the pressing body may have a flow hole that provides communication between the side on which the absorber is disposed and an inside of the tank on the side opposite to the side on which the absorber is disposed.

In some embodiments, the stopper may include a projection that projects toward the absorber that is inserted into the space inside the outer wall.

In some embodiments, the liquid collector may include a piston that is movable back and forth relative to the absorber in the support. In some embodiments, the piston may include a compressing member that compresses and deforms the absorber so that the liquid is discharged from the absorber into the tank. In some embodiments, a piston that is movable back and forth in the longitudinal direction in the tube of the support may be provided. In some embodiments, the piston may compress and deform the absorber in the tube so that the liquid is discharged from the absorber. In some embodiments, the piston may have a communication hole that provides communication between a side adjacent to the absorber and a side opposite to the side adjacent to the absorber.

In some embodiments, the absorber may be compressed in a direction along a plane crossing a pipe axis direction or the longitudinal direction. For example, the absorber may be squeezed in a direction crossing the pipe axis direction or the longitudinal direction, for example, in a sideways direction. For example, the absorber may be squeezed by the hand, fingers, or the like (for example, between the thumb and another finger) from both sides in a direction crossing the pipe axis direction or the longitudinal direction toward a central axis. For example, a cylinder may be made of a deformable material. The liquid absorbed in the absorber may be discharged due to compression or deformation as described above.

In some embodiments, the tank may have an air hole that provides communication with the outside. In some embodiments, the air hole allows passage of air between the inside and the outside of the tank when the amount of liquid contained is increased or when the absorber is compressed and deformed by the compressing member or the pressing body. Accordingly, the operation of collecting the liquid and the operation of compressing and deforming the absorber are not easily impeded by the internal pressure of the tank. In some embodiments, the air hole may be sealable. For example, a plug or seal may be provided to close the air hole from the outside of the container. The inner space can be sealed by closing the air hole. Thus, for example, without limitation, leakage of the liquid collected in the inner space to the outside can be prevented.

In some embodiments, the liquid collector may be configured such that the liquid absorbed in the absorber is discharged into the tank due to compression of air from the end of the support at which the absorber is disposed.

In some embodiments, the liquid collector may include a first cylinder that supports the absorber at an end of the first cylinder and in which a liquid-receiving space that receives the liquid is defined. In this case, in some embodiments, the liquid collector may include a second cylinder into which the first cylinder is insertable. In some embodiments, the second cylinder may be configured to press the absorber so that the liquid absorbed in the absorber moves into the liquid-receiving space when the first cylinder is inserted into the second cylinder.

In some embodiments, the liquid collector may include a first cylinder that supports the absorber at an end of the first cylinder. In some embodiments, the liquid collector may include a second cylinder into which the first cylinder is insertable. In some embodiments, a liquid-receiving space that receives the liquid may be defined in the second cylinder. In some embodiments, the second cylinder may be configured to compress and deform the absorber so that the liquid absorbed in the absorber moves into the liquid-receiving space when the first cylinder is inserted into the second cylinder.

In some embodiments, the first cylinder and the second cylinder may be closely fitted to each other such that the first cylinder and the second cylinder are movable relative to each other in the longitudinal direction. In some embodiments, a small gap may be provided between the first cylinder and the second cylinder. In some embodiments, the gap between the first cylinder and the second cylinder may be sealed.

In some embodiments, a method for collecting liquid may include causing an absorber to absorb liquid by bringing the absorber into contact with the liquid. In some embodiments, the method for collecting liquid may include causing the absorber to discharge the liquid absorbed in the absorber. In some embodiments, the method for collecting liquid may include receiving the liquid discharged from the absorber in a tank.

In some embodiments, in the method for collecting liquid, causing the absorber to discharge the liquid absorbed in the absorber includes compressing the absorber.

In some embodiments, the conditions of absorption of the liquid (for example, the amount of absorption and the status of a liquid-absorbing step (complete, incomplete, etc.)) may be determined based on a change in characteristics of the absorber.

In some embodiments, the amount of change in volume (for example, the amount of expansion) of the absorber may be used to determine the conditions of absorption of the liquid. The amount of expansion (length) of the absorber in the pipe axis direction may be used to measure the amount of the collected liquid, or to determine whether the absorbing step is completed. The amount of expansion of the absorber may be measured by using a scale mark provided on the support. For example, when the absorber expands inside the support, the amount of expansion may be measured by using the scale mark on the support. For example, the scale mark may be provided on an ordinary outer wall. When the absorber expands outside the support, the amount of expansion of the absorber may be measured by using a scale mark provided on an outer wall having a tubular shape. For example, a separate scale may be prepared, and the amount of expansion of the absorber may be measured by placing the scale on the absorber.

In some embodiments, the amount of absorption of the liquid may be determined based on a change in color of the absorber. For example, the absorber may include a substance that changes color when the substance comes into contact with water. The amount of absorption of the liquid may be determined based on the size (for example, length in the pipe axis direction) of a portion of the absorber whose color is changed. The increase in length may be measured by using, for example, a scale mark provided on the support. For example, it may be determined that a sufficient amount of liquid is collected when the color of the entirety of the absorber or the entirety of a certain portion of the absorber is changed.

In some embodiments, a plurality of absorbers that are connected to each other may be provided, and the amount of absorption of the liquid may be determined based on the number of absorbers whose characteristics are changed. For example, it may be determined that a sufficient amount of liquid is collected when a change in characteristics (for example, expansion or change in color) is completed in the first and second absorbers counted from an end (end in contact with or close to the target) and started in the third absorber.

In some embodiments, the tank may have a scale mark. The scale mark may be used to measure the amount of liquid collected in the tank, or to determine whether a sufficient amount of liquid is collected.

At least a portion or the entirety of the tank may be made of a material having transparency characteristics that allow optical observation of the inside of the tank from the outside. Examples of the material having transparency characteristics that allow optical observation of the inside of the tank from the outside include, but are not limited to, resin materials and glass materials. The material having transparency characteristics that allow optical observation of the inside of the tank from the outside may have transparency characteristics such that the amount of liquid accumulated in the tank is observable visually or with a camera from the outside of the tank.

In some embodiments, the method for collecting liquid may further include providing the above-described liquid collector.

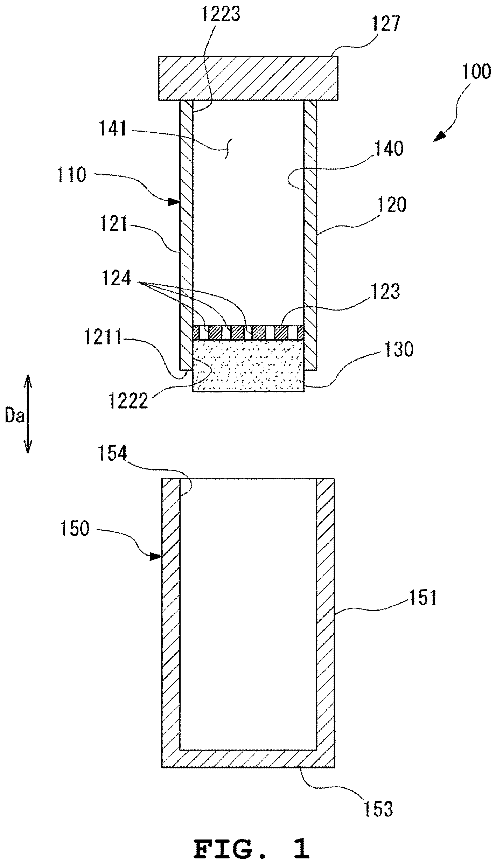

Figs. 1 to 3 illustrate a liquid collector 100 according to an embodiment. As illustrated in Fig. 1, the liquid collector 100 includes a collector body 110 and a compressing member 150 that serves as a second cylinder.

The collector body 110 includes a support 120 that serves as a first cylinder, an absorber 130, and a tank 140.

The support 120 supports the absorber 130. The support 120 has a tubular shape that extends in a longitudinal direction Da.

The support 120 includes a tube 121 that extends in the longitudinal direction Da and a stopper (fixing portion) 123. The tube 121 has, for example, one of a circular shape, an elliptical shape, a polygonal shape, etc., in cross-section when viewed in the longitudinal direction Da.

The stopper 123 is provided at one end of the tube 121 in the longitudinal direction Da. The stopper 123 is disposed in the tube 121. The stopper 123 extends along a plane substantially orthogonal to the longitudinal direction Da. The stopper 123 has a plurality of flow holes 124 that provide communication between both sides of the stopper 123 in the longitudinal direction Da. The stopper 123 may be obtained by forming the flow holes 124 in a plate member provided to extend along a plane substantially orthogonal to the longitudinal direction Da. The stopper 123 may be a grid material or a mesh material provided to extend along a plane substantially orthogonal to the longitudinal direction Da. When the absorber 130 is pressed against the stopper 123, the absorber 130 abuts against the stopper 123 so that movement of the absorber 130 in the longitudinal direction Da is restricted. The stopper 123 positions the absorber 130 with respect to the support 120 when the absorber 130 is pressed against the stopper 123 to discharge the liquid absorbed in the absorber 130 from the absorber 130.

The stopper 123 is displaced from a tube end 1211 of the tube 121 at one side in the longitudinal direction Da toward the other side in the longitudinal direction Da. Accordingly, the support 120 has a recess 1222 surrounded by the tube 121 and the stopper 123 at one end thereof in the longitudinal direction Da. The recess 1222 opens toward the one side in the longitudinal direction Da.

In some embodiments, the stopper 123 may include a projection or a three-dimensional structure described below that projects toward the absorber 130. According to this projection, for example, the absorber 130 can be further compressed and deformed so that the liquid can be more efficiently discharged from the absorber 130.

The tube 121 having the stopper 123 at one end thereof in the longitudinal direction Da has an opening 1223 at the other end in the longitudinal direction Da. A closing body 127 is disposed at the other end of the support 120 in the longitudinal direction Da. The closing body 127 is removably attachable to the opening 1223, or is capable of covering and uncovering the opening 1223.

The absorber 130 is supported by the support 120. The absorber 130 is disposed at one end of the support 120 in the longitudinal direction Da. The absorber 130 is capable of absorbing liquid that is in contact therewith.

The absorber 130 includes an elastic material. The entirety of the absorber 130 may be made of the elastic material. A portion of the absorber 130 may be made of the elastic material. The absorber 130 is elastically deformable when brought into contact with a target from which liquid is to be collected. The elastic material included in the absorber 130 may be a material that is both elastic and porous. Such an elastic material may be, for example, cellulose. The elastic material included in the absorber 130 may be a material other than cellulose (non-cellulose material).

The absorber 130 is fixed to the support 120. At least a portion of the absorber 130 is fixed to the support 120. In some embodiments, the absorber 130 may be fitted to the recess 1222 in the support 120 and be substantially supported by or fixed to an inner wall.

At least a portion of the absorber 130 is placed in the recess 1222. The absorber 130 may be disposed such that only a portion thereof that is adjacent to the support 120 is placed in the recess 1222, or such that the entirety thereof is placed in the recess 1222. The absorber 130 may be fixed to the tube 121 at the inner wall of the recess 1222. The absorber 130 may, for example, be spaced from the stopper 123 in the longitudinal direction Da. Alternatively, the absorber 130 may be joined to the stopper 123 by, for example, adhesion or fusion bonding.

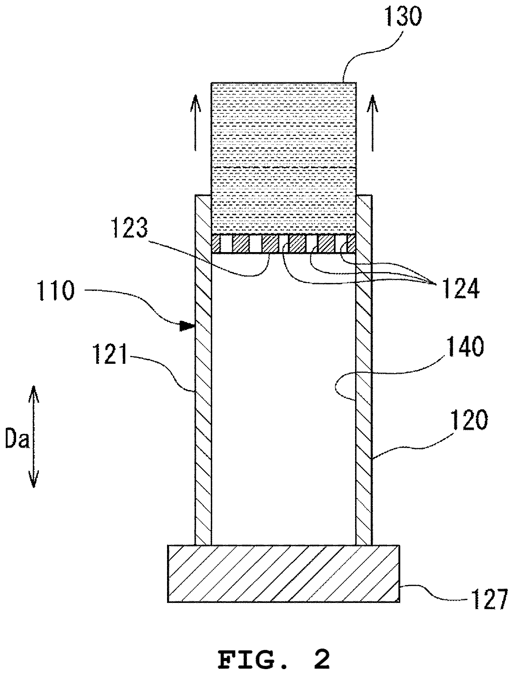

The absorber 130 is elastically deformable when pressed against a target subject or a target object, and absorbs liquid that is in contact therewith. As illustrated in Fig. 2, when the absorber 130 absorbs liquid, the absorber 130 expands to project from the support 120 mainly in the longitudinal direction Da.

The tank 140 receives liquid discharged from the absorber 130 when the absorber 130 is compressed. In some embodiments, an inside of the tube 121 of the support 120 serves as the tank 140. The tank 140 defines a liquid-receiving space 141 between the stopper 123 and the closing body 127 in the tube 121. The liquid-receiving space 141 is capable of receiving the liquid therein.

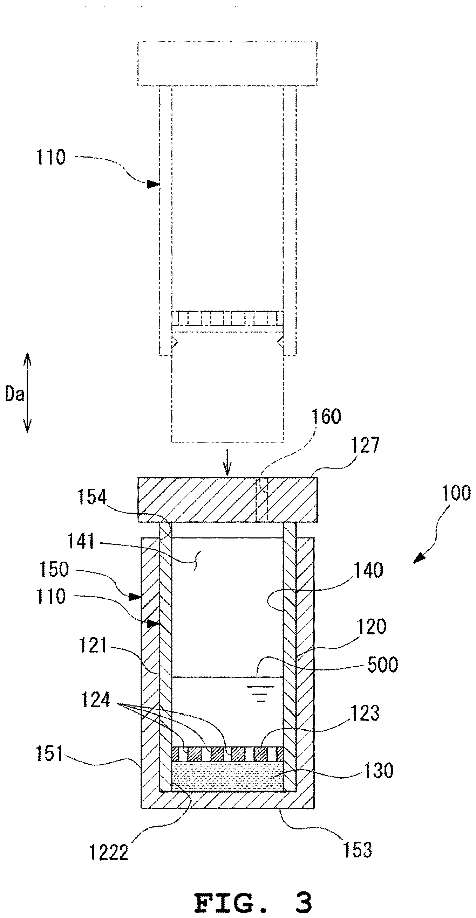

The compressing member 150 has a tubular shape with a bottom. The compressing member 150 includes an outer wall 151 having a tubular shape and a pressing plate 153 that are integrated together. The outer wall 151 having a tubular shape extends in the longitudinal direction Da. The pressing plate 153 is disposed at an end of the outer wall 151 at one side in the longitudinal direction Da. The pressing plate 153 extends along a plane substantially orthogonal to the longitudinal direction Da of the outer wall 151. The pressing plate 153 blocks the end of the outer wall 151 at the one side in the longitudinal direction Da. The outer wall 151 has an opening 154 at an end opposite to the end at the one side in the longitudinal direction Da at which the pressing plate 153 is provided. The support 120, which serves as a first cylinder, and the absorber 130 are insertable into the space inside the outer wall 151 from the opening 154 toward the pressing plate 153. As illustrated in Fig. 3, the support 120 having the absorber 130 at an end thereof is insertable into the space inside the outer wall 151 of the compressing member 150. The compressing member 150 is movable relative to the absorber 130 in the longitudinal direction Da.

As illustrated in Fig. 3, when the support 120 is inserted into the space inside the outer wall 151, the absorber 130 provided at the end of the support 120 comes into contact with the pressing plate 153 of the compressing member 150. When the support 120 is pushed further inward, the absorber 130 is pressed by the pressing plate 153. Accordingly, the absorber 130 is compressed and deformed so that liquid 500 absorbed in the absorber 130 is discharged. The liquid 500 discharged from the absorber 130 passes through the flow holes 124 formed in the stopper 123 and moves into the liquid-receiving space 141 of the tank 140 formed in the support 120. Thus, the liquid 500 absorbed in the absorber 130 is collected in the tank 140. The pressing plate 153 may have a projection or a projecting structure capable of entering the recess 1222 in the support 120. Thus, the absorber 130 can be efficiently compressed.

In some embodiments, the absorber may be maintained in a compressed state. In some embodiments, the relative position between the collector body and the compressing member or between the support and the compressing member may be fixed while the absorber is compressed. For example, the collector body and the compressing member may be fitted to each other, or the support and the compressing member may be fitted to each other. For example, they may be removably or non-removably fitted to each other. For example, they may be mechanically fitted to each other.

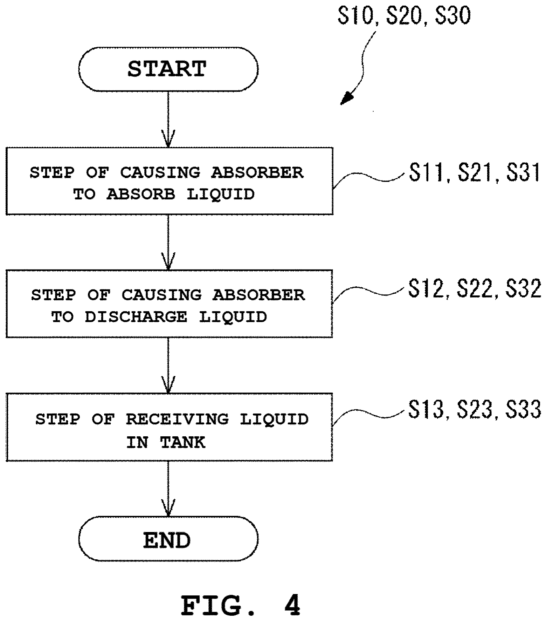

Fig. 4 is a flowchart of a liquid collection method S10 according to an embodiment. As illustrated in Fig. 4, the liquid collection method S10 includes a step S11 of causing the absorber 130 to absorb the liquid 500, a step S12 of causing the absorber 130 to discharge the liquid 500, and a step S13 of receiving the liquid 500 in the tank 140.

In the step S11 of causing the absorber 130 to absorb the liquid 500, the absorber 130 of the collector body 110 is brought into contact with the liquid 500 (illustrated in Fig. 3 and other figures, hereinafter omitted). In some embodiments, when the liquid collector 100 is used to collect body fluid as the liquid 500, the absorber 130 is directly pressed against or brought into contact with a part of the target subject in which the body fluid is secreted. Examples of the part of the target subject against which the absorber 130 is directly pressed and in which the body fluid is secreted include, but are not limited to, an eyeball, regions around the eye at the inner and outer corners of the eye, a part in the oral cavity (for example, a sublingual region), a part in the nasal cavity, and the skin surface. In some embodiments, the body fluid secreted in the target subject may be contained in a container. In such a case, the absorber 130 may be brought into contact with the body fluid contained in the container so that the liquid 500 is absorbed therein. In some embodiments, the support 120 may be positioned such that the end at which the absorber 130 is provided faces upward. The body fluid may be dropped onto the absorber 130 from above.

As illustrated in Fig. 2, the absorber 130 expands when the liquid 500 is absorbed therein. In some embodiments, the degree of expansion of the absorber 130 may be visually observed to check or measure the amount of the liquid 500 that is absorbed (collected).

In the step S12 of causing the absorber 130 to discharge the liquid 500, as illustrated in Fig. 3, the support 120 supporting the absorber 130 in which the liquid 500 is absorbed is inserted into the space inside the outer wall 151 of the compressing member 150 having a tubular shape with a bottom from the opening 154. When the support 120 is further inserted into the space inside the outer wall 151 of the compressing member 150, the absorber 130 is pushed against the pressing plate 153. The absorber 130 is pressed by the pressing plate 153. Accordingly, the absorber 130 is compressed and deformed. The liquid 500 absorbed in the absorber 130 is discharged.

In the step S13 of receiving the liquid 500 in the tank 140, the liquid 500 discharged from the absorber 130 passes through the flow holes 124 formed in the stopper 123 and moves into the liquid-receiving space 141 of the tank 140 formed in the support 120. Thus, the liquid 500 absorbed in the absorber 130 is collected (received) in the tank 140.

In some embodiments, when the liquid 500 flows into the liquid-receiving space 141 of the tank 140, the pressure in the liquid-receiving space 141 increases. Accordingly, in some embodiments, as illustrated in Fig. 3, the outer wall 151 or the closing body 127 may have an air hole 160. Thus, the increase in pressure in the liquid-receiving space 141 due to the liquid 500 flowing into the liquid-receiving space 141 can be suppressed. In some embodiments, the liquid may be extracted from the liquid-receiving space 141 or a measurement reagent or the like may be introduced into the liquid-receiving space 141 through, for example, the outer wall 151, the closing body 127, or the air hole 160. For example, the closing body 127 may have a structure that enables a hole to be formed therein, such as a septum or a structure with radial slits (structure of a garbage disposal splash guard).

In the above-described liquid collector 100, the absorber 130 including the elastic material absorbs the liquid 500 in contact with the absorber 130. Accordingly, the absorber 130 is capable of efficiently collecting the liquid 500. The absorber 130 is elastically deformable when brought into contact with a target subject or a target object. Accordingly, the liquid 500 can be collected with less stress on the target subject or the target object.

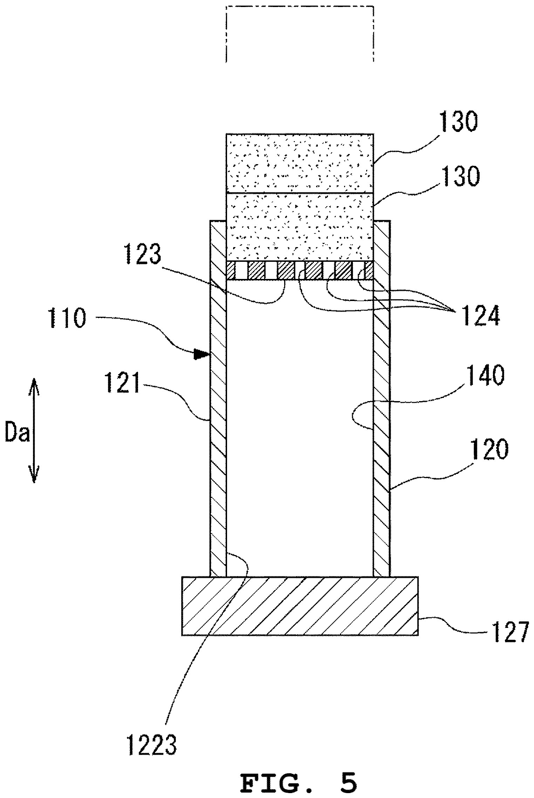

In some embodiments, as illustrated in Fig. 5, a plurality of absorbers 130 may be stacked together. In the structure of the present disclosure illustrated in Fig. 5, two absorbers 130 are stacked together. In some embodiments, three or more absorbers 130 may be stacked together. In some embodiments, the absorbers 130 that are stacked together may have the same color and shape and be made of the same material. In some embodiments, at least one of the characteristics, such as color, shape, and material, may differ between the absorbers 130 that are stacked together. By utilizing the difference in characteristics, such as color, shape, and material, between the absorbers 130, the degree of absorption of liquid 500 may be easily visually checked based on, for example, a change or difference in characteristics, such as a change in color or expansion, caused when the liquid 500 is absorbed in the absorber 130. When the absorbers 130 are stacked together so that the overall height of the absorbers 130 in the longitudinal direction Da is increased, a larger amount of liquid 500 can be absorbed. In some embodiments, a single absorber 130 may be formed so that the height thereof is equivalent to the overall height of a plurality of absorbers 130 stacked together. Accordingly, a larger amount of liquid 500 can be absorbed.

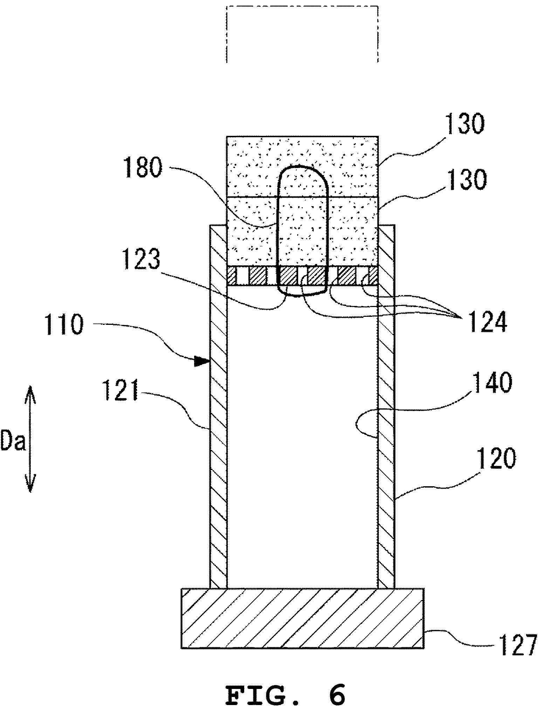

In some embodiments, as illustrated in Fig. 6, the absorbers 130 that are stacked together may be connected to the support 120 with a line-shaped connecting member 180. Examples of the line-shaped connecting member 180 include, but are not limited to, a thread, a string, a cable, and a wire.

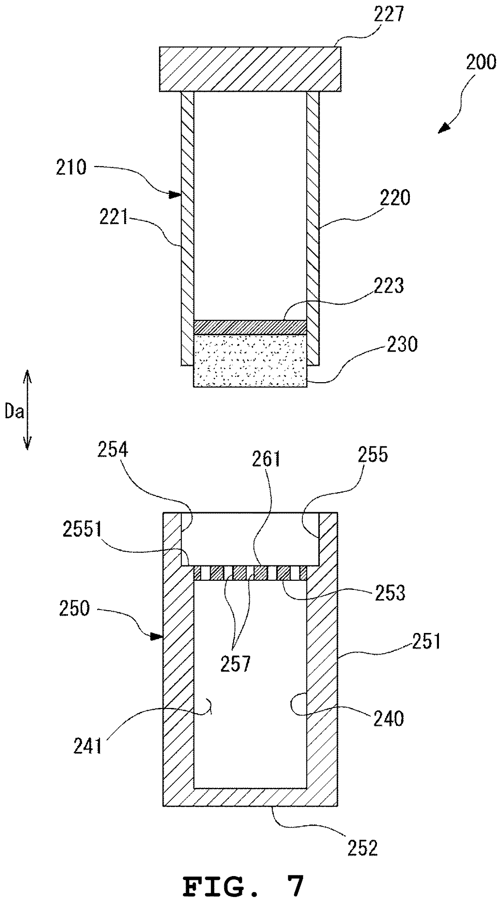

In some embodiments, the collector body for collecting the liquid 500 may be a member separate from the absorber and the support for supporting the absorber. Fig. 7 to 10 illustrate a liquid collector 200 according to an embodiment.

The liquid collector 200 illustrated in Fig. 7 includes a collector body 210 and a compressing member 250 that serves as a second cylinder.

The collector body 210 includes a support 220 that serves as a first cylinder and an absorber 230. The support 220 supports the absorber 230. The support 220 has a tubular shape that extends in a longitudinal direction Da. The support 220 includes a tube 221 that extends in the longitudinal direction Da and a stopper 223. The tube 221 has, for example, one of a circular shape, an elliptical shape, a polygonal shape, etc., in cross-section when viewed in the longitudinal direction Da. In some embodiments, the support 220 may have a shaft shape.

The stopper 223 is provided at one end of the tube 221 of the support 220 in the longitudinal direction Da. The stopper 223 is disposed in the tube 221. The stopper 223 extends along a plane substantially orthogonal to the longitudinal direction Da. When the absorber 230 is pressed against the stopper 223, the absorber 230 abuts against the stopper 223 so that movement of the absorber 230 is restricted. The stopper 223 positions the absorber 230 with respect to the support 220.

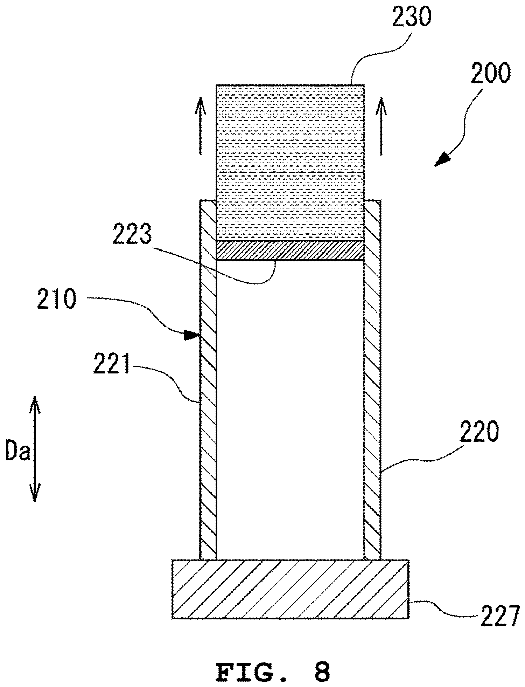

The absorber 230 is supported by the support 220. The absorber 230 is disposed at one end of the support 220 in the longitudinal direction Da. The absorber 230 is capable of absorbing liquid 500 that is in contact therewith. As illustrated in Fig. 8, the absorber 230 expands when the absorber 230 comes into contact with and absorbs the liquid 500. At least a portion of the absorber 230 is fixed to the support 220. The absorber 230 is elastically deformable when pressed against a target subject or a target object, and absorbs the liquid 500 that is in contact therewith. As illustrated in Fig. 8, when the absorber 230 absorbs the liquid 500, the absorber 230 expands to project from the support 220 mainly in the longitudinal direction Da.

As illustrated in Fig. 7, the compressing member 250 is separate from the collector body 210. The collector body 210 is attachable to the compressing member 250. The compressing member 250 has a tubular shape with a bottom. The compressing member 250 includes an outer wall 251 having a tubular shape, a bottom plate 252, and a pressing body 253 that are integrated together. The outer wall 251 having a tubular shape extends in the longitudinal direction Da. The bottom plate 252 is disposed at an end of the outer wall 251 at one side in the longitudinal direction Da. The bottom plate 252 extends along a plane substantially orthogonal to the longitudinal direction Da of the outer wall 251. The bottom plate 252 blocks the end of the outer wall 251 at the one side in the longitudinal direction Da. The outer wall 251 has an opening 254 at an end opposite to the end at the one side in the longitudinal direction Da at which the bottom plate 252 is provided. The support 220, which serves as a first cylinder, and the absorber 230 are insertable into the space inside the outer wall 251 from the opening 254 toward the pressing body 253. Alternatively, the outer wall 251 is formed to allow the insertion. The support 220 is insertable into the space inside the outer wall 251 of the compressing member 250. The compressing member 250 is movable relative to the absorber 230 in the longitudinal direction Da.

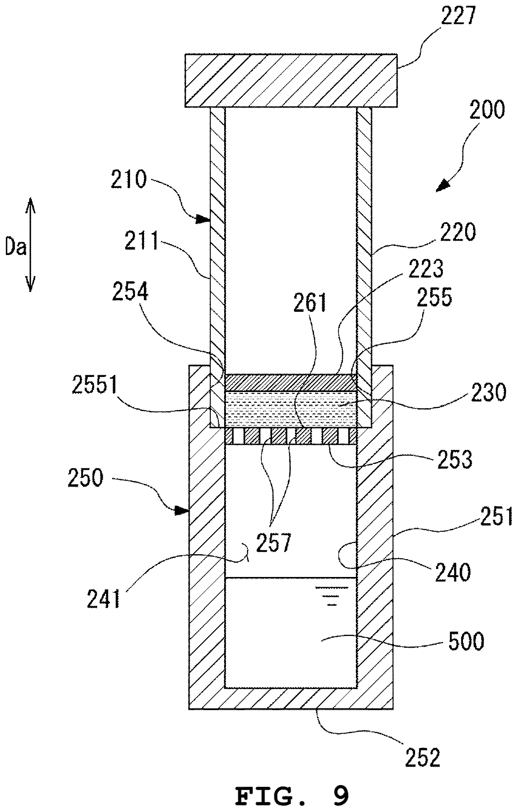

An inner peripheral surface of the outer wall 251 has a large-diameter portion 255 at an end of the compressing member 250 at the other side in the longitudinal direction Da. The large-diameter portion 255 has an inner diameter greater than an inner diameter of the outer wall 251 at an end of the compressing member 250 at the one side in the longitudinal direction Da. A step portion 2551 that is orthogonal to the longitudinal direction Da is formed at an end of the large-diameter portion 255 at the one side in the longitudinal direction Da. As illustrated in Fig. 9, the support 220 that serves as a first cylinder is inserted into the large-diameter portion 255 through the opening 254 of the compressing member 250. The tube 221 of the support 220 comes into contact with the step portion 2551 so that a depth of insertion into the compressing member 250 is restricted.

As illustrated in Fig. 7, the pressing body 253 is disposed inside the outer wall 251 of the compressing member 250. The pressing body 253 is disposed to extend along a plane substantially orthogonal to the longitudinal direction Da. The pressing body 253 has a surface 261 that faces the opening 254 and that is disposed at substantially the same position as the position of the step portion 2551 of the large-diameter portion 255 in the longitudinal direction Da. As illustrated in Fig. 9, the pressing body 253 is disposed to face the absorber 230 in the longitudinal direction Da when the absorber 230 (support 220) is inserted into the space inside the outer wall 251 of the compressing member 250 from the opening 254.

The pressing body 253 presses the absorber 230 when the support 220 is pushed toward the one side in the longitudinal direction Da.

The pressing body 253 has communication holes 257 that provide communication between both sides of the pressing body 253 in the longitudinal direction Da, that is, between the side on which the absorber 230 is provided and the side opposite thereto.

In the embodiment of the present disclosure, as illustrated in Fig. 7, a tank 240 is provided in the compressing member 250.

The tank 240 receives the liquid 500 discharged from the absorber 230 when the absorber 230 is compressed. The tank 240 is formed in the space inside the outer wall 251 of the compressing member 250. The tank 240 is disposed on a side of the pressing body 253 opposite to the side on which the absorber 230 is disposed. The tank 240 defines a liquid-receiving space 241 between the bottom plate 252 and the pressing body 253 inside the outer wall 251. The liquid-receiving space 241 is capable of receiving the liquid 500 therein.

As illustrated in Fig. 4, a liquid collection method S20 includes a step S21 of causing the absorber 230 to absorb the liquid 500, a step S22 of causing the absorber 230 to discharge the liquid 500, and a step S23 of receiving the liquid 500 in the tank 240.

In the step S21 of causing the absorber 230 to absorb the liquid 500, the absorber 230 of the collector body 210 is brought into contact with the liquid 500. As illustrated in Fig. 8, the absorber 230 expands when the liquid 500 is absorbed therein.

In the step S22 of causing the absorber 230 to discharge the liquid 500, as illustrated in Fig. 9, the support 220 supporting the absorber 230 in which the liquid 500 is absorbed is inserted into the space inside the outer wall 251 of the compressing member 250 from the opening 254. The support 220 is further inserted into the space inside the outer wall 251, and the absorber 230 is pushed against the pressing body 253. The absorber 230 is pressed by the pressing body 253. The absorber 230 is compressed and deformed in the longitudinal direction Da. Accordingly, the liquid 500 absorbed in the absorber 230 is discharged.

In the step S23 of receiving the liquid 500 in the tank 240, the liquid 500 discharged from the absorber 230 passes through the communication holes 257 formed in the pressing body 253 and moves into the liquid-receiving space 241 of the tank 240. Thus, the liquid 500 absorbed in the absorber 230 is collected (received) in the tank 240. The pressing body 253 may have a projection or a projecting structure capable of entering the support 220. Thus, the absorber 230 can be efficiently compressed.

The above-described liquid collector 200 is capable of efficiently collecting the liquid 500.

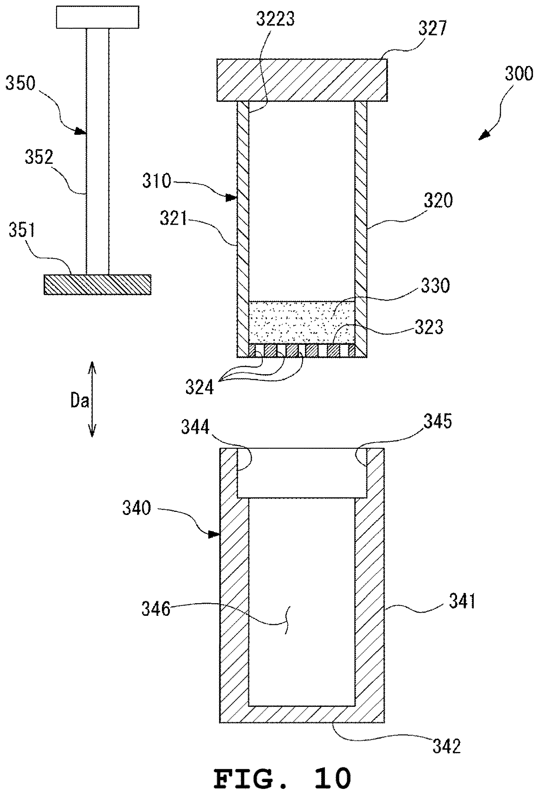

In some embodiments, the absorber may expand inward in the support when the liquid 500 is absorbed therein. Figs. 10 to 12 illustrate a liquid collector 300 according to an embodiment. As illustrated in Fig. 10, the liquid collector 300 includes a collector body 310, a tank 340 that serves as a second cylinder, and a compressing member 350.

The collector body 310 includes a support 320 that serves as a first cylinder and an absorber 330.

The support 320 supports the absorber 330. The support 320 has a tubular shape that extends in a longitudinal direction Da. The support 320 includes a tube 321 that extends in the longitudinal direction Da and a stopper 323. The tube 321 has, for example, one of a circular shape, an elliptical shape, a polygonal shape, etc., in cross-section when viewed in the longitudinal direction Da.

The stopper 323 is provided at one end of the tube 321 in the longitudinal direction Da. The stopper 323 is disposed in the tube 321. The stopper 323 extends along a plane substantially orthogonal to the longitudinal direction Da. The stopper 323 has a plurality of flow holes 324 that provide communication between both sides of the stopper 323 in the longitudinal direction Da. The stopper 323 may be obtained by forming the flow holes 324 in a plate member provided to extend along a plane substantially orthogonal to the longitudinal direction Da. The stopper 323 may be a grid material or a mesh material provided to extend along a plane substantially orthogonal to the longitudinal direction Da. When the absorber 330 is pressed against the stopper 323, the absorber 330 abuts against the stopper 323 so that further movement thereof is restricted. The stopper 323 positions the absorber 330 with respect to the support 320.

The tube 321 having the stopper 323 at one end thereof in the longitudinal direction Da has an opening 3223 at the other end in the longitudinal direction Da. A closing body 327 is disposed at the other end of the support 320 in the longitudinal direction Da. The closing body 327 is removably attachable to the opening 3223 or is capable of covering and uncovering the opening 3223.

The absorber 330 is disposed in the tube 321 of the support 320. The absorber 330 is disposed at one end of the support 320 in the longitudinal direction Da. In the embodiment of the present disclosure, the absorber 330 is disposed in the tube 321 at the end of the support 320 at which the stopper 323 is provided in the longitudinal direction Da. In the embodiment of the present disclosure, the absorber 330 may instead be disposed at an end of the support 320 opposite to the end at which the stopper 323 is provided in the longitudinal direction Da.

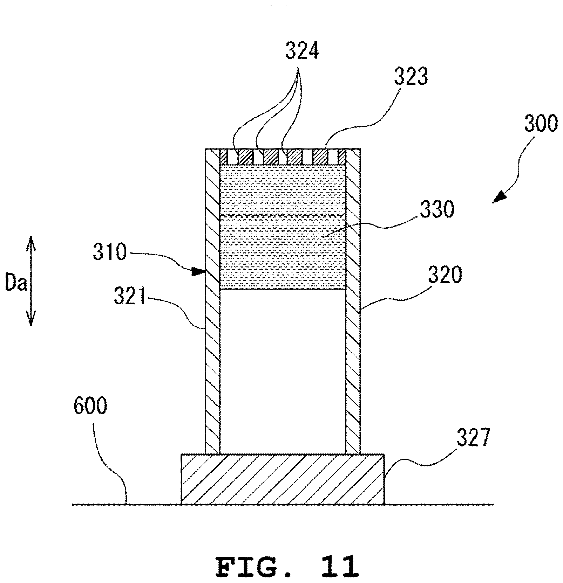

The absorber 330 is capable of absorbing the liquid 500 that is in contact therewith. The absorber 330 is expandable in the support 320 when the liquid 500 is absorbed therein. The absorber 330 is at least partially fixed to the support 320. As illustrated in Fig. 11, the absorber 330 is expandable in the longitudinal direction Da in the tube 321 when the liquid 500 is absorbed therein. At least a portion of the absorber 330 is fixed to the absorber 330. The absorber 330 may be joined to the stopper 323 by, for example, adhesion or fusion bonding.

The absorber 330 includes an elastic material. The entirety of the absorber 330 may be made of the elastic material. A portion of the absorber 330 may be made of the elastic material. The absorber 330 is elastically deformable when brought into contact with a target from which the liquid 500 is to be collected. The elastic material included in the absorber 330 may be a material that is both elastic and porous. Such an elastic material may be, for example, cellulose. The elastic material included in the absorber 330 may be a material other than cellulose.

The tank 340 is separate from the collector body 310. The tank 340, which serves as a second cylinder, is attachable to an end of the support 320 at which the absorber 330 and the stopper 323 are disposed. The tank 340 has a tubular shape with a bottom. The tank 340 includes a tubular wall 341 and a bottom plate 342 that are integrated together. The tubular wall 341 having a tubular shape extends in the longitudinal direction Da. The bottom plate 342 is disposed at an end of the tubular wall 341 at one side in the longitudinal direction Da. The bottom plate 342 extends along a plane substantially orthogonal to the longitudinal direction Da of the tubular wall 341. The bottom plate 342 blocks the end of the tubular wall 341 at the one side in the longitudinal direction Da. A liquid-receiving space 346 is defined in the tank 340. The tank 340 is configured such that the liquid 500 discharged from the absorber 330 when the absorber 330 is compressed can be received by the liquid-receiving space 346.

The tubular wall 341 has an opening 344 at an end opposite to the end at the one side in the longitudinal direction Da at which the bottom plate 342 is provided. An inner peripheral surface of the tubular wall 341 has a large-diameter portion 345 at an end of the tank 340 at the other side in the longitudinal direction Da. The large-diameter portion 345 has an inner diameter greater than an inner diameter of the tubular wall 341 at an end of the tank 340 at the one side in the longitudinal direction Da. The support 320, which serves as a first cylinder, is insertable into the space inside the tubular wall 341 from the opening 344 (large-diameter portion 345).

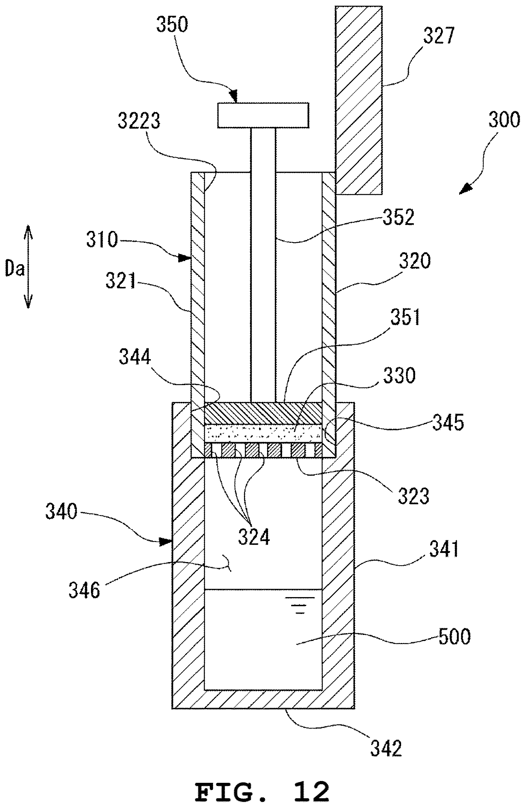

The compressing member 350 is separate from the collector body 310 and the tank 340. The compressing member 350 includes a piston 351 and a support shaft 352 that are integrated together. Referring to Fig. 12, the piston 351 is movable back and forth in the longitudinal direction Da in the tube 321 of the support 320. The piston 351 moves back and forth in the longitudinal direction Da such that an outer peripheral surface thereof slides along an inner peripheral surface of the tube 321.

The support shaft 352 has one end fixed to the piston 351. When the support shaft 352 is inserted in the tube 321, the support shaft 352 extends in the longitudinal direction Da of the tube 321, and the other end of the support shaft 352 projects from the opening 3223 of the tube 321.

When an operator pushes the support shaft 352 toward the absorber 330 in the longitudinal direction Da, the compressing member 350 compresses and deforms the absorber 330 in the tube 321 with the piston 351. Thus, the liquid 500 is discharged from the absorber 330.

As illustrated in Fig. 4, a liquid collection method S30 includes a step S31 of causing the absorber 330 to absorb the liquid 500, a step S32 of causing the absorber 330 to discharge the liquid 500, and a step S33 of receiving the liquid 500 in the tank 340.

In the step S31 of causing the absorber 330 to absorb the liquid 500, the absorber 330 of the collector body 310 is brought into contact with the liquid 500. In some embodiments, the liquid 500 is introduced into the liquid-receiving space 346 of the tank 340 provided in the support 320 through the flow holes 324 formed in the stopper 323 of the collector body 310. In some embodiments, as illustrated in Fig. 11, the closing body 327 is placed on a support surface 600 so that the stopper 323 having the flow holes 324 faces upward. In this state, the liquid 500 may be introduced into the liquid-receiving space 346 through the flow holes 324 from above. Alternatively, the end of the collector body 310 at which the stopper 323 is provided may be immersed in the liquid 500 collected in, for example, a container, and the liquid 500 may be introduced into the liquid-receiving space 346 through the flow holes 324.

The liquid 500 introduced into the liquid-receiving space 346 is absorbed by the absorber 330 disposed in the liquid-receiving space 346. As illustrated in Fig. 11, the absorber 330 expands in the longitudinal direction Da in the tube 321 when the liquid 500 is absorbed therein.

In the step S32 of causing the absorber 330 to discharge the liquid 500, as illustrated in Fig. 12, the support 320 supporting the absorber 330 in which the liquid 500 is absorbed is inserted into the opening 344 in the tank 340 having a tubular shape with a bottom. The compressing member 350 is pushed inward so that the piston 351 pushes the absorber 330 against the stopper 323 in the tube 321 of the support 320. The absorber 330 is pressed by the piston 351. The absorber 330 is compressed and deformed. Thus, the liquid 500 absorbed in the absorber 330 is discharged through the flow holes 324 in the stopper 323.

In the step S33 of receiving the liquid 500 in the tank 340, the liquid 500 discharged from the absorber 330 passes through the flow holes 324 formed in the stopper 323 and moves into the liquid-receiving space 346 of the tank 340 formed in the support 320. Thus, the liquid 500 absorbed in the absorber 330 is collected (received) in the tank 340.

The above-described liquid collector 300 is capable of efficiently collecting the liquid 500.

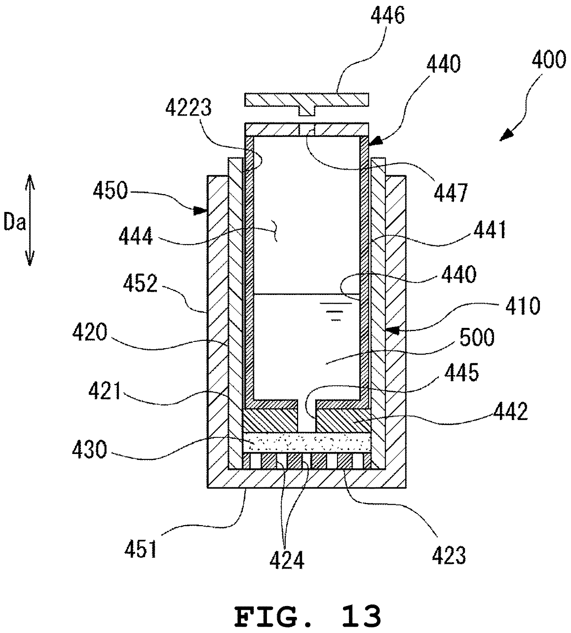

In some embodiments, the absorber may expand in the support when the liquid 500 is absorbed therein, and the absorbed liquid 500 may be collected in the support. Fig. 13 illustrates a liquid collector 400 according to an embodiment. As illustrated in Fig. 13, the liquid collector 400 includes a collector body 410 and a tank 440 that serves as a second cylinder.

The collector body 410 includes a support 420 that serves as a first cylinder and an absorber 430.

The support 420 supports the absorber 430. The support 420 has a tubular shape that extends in a longitudinal direction Da. The support 420 includes a tube 421 that extends in the longitudinal direction Da and a stopper 423. The tube 421 has, for example, one of a circular shape, an elliptical shape, a polygonal shape, etc., in cross-section when viewed in the longitudinal direction Da.

The stopper 423 is provided at one end of the tube 421 in the longitudinal direction Da. The stopper 423 is disposed in the tube 421. The stopper 423 extends along a plane substantially orthogonal to the longitudinal direction Da. The stopper 423 has a plurality of flow holes 424 that provide communication between both sides of the stopper 423 in the longitudinal direction Da. The stopper 423 may be obtained by forming the flow holes 424 in a plate member provided to extend along a plane substantially orthogonal to the longitudinal direction Da. The stopper 423 may be a grid material or a mesh material provided to extend along a plane substantially orthogonal to the longitudinal direction Da. When the absorber 430 is pressed against the stopper 423, the absorber 430 abuts against the stopper 423 so that further movement thereof is restricted. The stopper 423 positions the absorber 430 with respect to the support 420.

The tube 421 having the stopper 423 at one end thereof in the longitudinal direction Da has an opening 4223 at the other end in the longitudinal direction Da.

The absorber 430 is disposed in the tube 421 of the support 420. The absorber 430 is disposed at one end of the support 420 in the longitudinal direction Da. In the embodiment of the present disclosure, the absorber 430 is disposed in the tube 421 at the end of the support 420 at which the stopper 423 is provided in the longitudinal direction Da. In the embodiment of the present disclosure, the absorber 430 may instead be disposed at an end of the support 420 opposite to the end at which the stopper 423 is provided in the longitudinal direction Da.

The absorber 430 is capable of absorbing the liquid 500 that is in contact therewith. The absorber 430 is expandable in the support 420 when the liquid 500 is absorbed therein. The absorber 430 is at least partially fixed to the support 420. The absorber 430 is expandable in the longitudinal direction Da in the tube 421 when the liquid 500 is absorbed therein. At least a portion of the absorber 430 is fixed to the absorber 430. The absorber 430 may be joined to the stopper 423 by, for example, adhesion or fusion bonding.

The absorber 430 includes an elastic material. The entirety of the absorber 430 may be made of the elastic material. A portion of the absorber 430 may be made of the elastic material. The absorber 430 is elastically deformable when brought into contact with a target from which the liquid 500 is to be collected. The elastic material included in the absorber 430 may be a material that is both elastic and porous. Such an elastic material may be, for example, cellulose. The elastic material included in the absorber 430 may be a material other than cellulose.

In some embodiments, the tank 440 may be used. The tank 440 is separate from the collector body 410. The tank 440 includes a tank body 441 and a piston 442. The tank body 441 has a hollow tubular shape, and a liquid-receiving space 444 capable of receiving the liquid 500 is defined therein. The tank body 441 is insertable into the tube 421 of the support 420 of the collector body 410. The piston 442 is disposed at an end of the tank body 441 at one side in the longitudinal direction Da. The piston 442 is movable back and forth in the longitudinal direction Da in the tube 421 of the support 420. The piston 442 moves back and forth in the longitudinal direction Da such that an outer peripheral surface thereof slides along an inner peripheral surface of the tube 421. The piston 442 compresses and deforms the absorber 430 in the tube 421. The piston 442 has a communication hole 445 that provides communication between the absorber 430 and the liquid-receiving space 444 in the tank body 441 in the longitudinal direction Da.

The tank body 441 has an air hole 447 at an end opposite to the end at which the piston 442 is provided in the longitudinal direction Da. A cap 446 capable of closing the air hole 447 is attachable to the tank body 441. In some embodiments, the tank body 441 may have no air hole 447, and may be sealed with a removable cap 446.

To collect the liquid 500 from the absorber 430 by using the tank 440, an outer cover 450 is attached to the support 420. The outer cover 450 blocks the flow holes 424 in the stopper 423 of the support 420. The outer cover 450 includes a closing body 451 that blocks the flow holes 424 in the stopper 423 and a tubular wall 452 that extends from the closing body 451 toward the other side in the longitudinal direction Da. The support 420 is inserted into the outer cover 450. The closing body 451 is pressed against the stopper 423 to block the flow holes 424.

To collect the liquid 500 from the absorber 430 in the step S32 of causing the absorber 430 to discharge the liquid 500 in Fig. 4, the tank 440 is inserted into the tube 421 from the opening 4223 of the support 420, as illustrated in Fig. 13. Thus, the inside of the support 420 serves as the tank 440. The tank 440 is pushed inward so that the piston 442 pushes the absorber 430 against the stopper 423 in the tube 421 of the support 420. The absorber 430 is pressed by the piston 442. The absorber 430 is compressed and deformed in the longitudinal direction Da. Accordingly, the liquid 500 absorbed in the absorber 430 moves into the tank body 441 of the tank 440 through the communication hole 445 in the piston 442. Thus, the liquid 500 absorbed in the absorber 430 is collected (received) in the tank 440.

The above-described liquid collector 400 is capable of efficiently collecting the liquid 500.

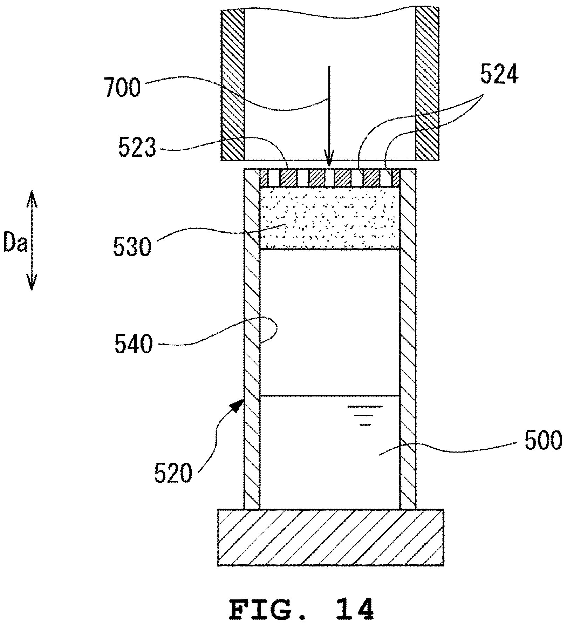

In some embodiments, the liquid 500 absorbed in the absorber may be collected without compressing the absorber. As illustrated in Fig. 14, air 700 may be compressed and introduced into a support 520 from an end of the support 520 at which an absorber 530 is disposed through flow holes 524 in a stopper 523. Thus, the liquid 500 absorbed in the absorber 530 is discharged into a tank 540 provided in the support 520.

The present disclosure also provides the following embodiments:
A001 A liquid collector including:
   an absorber including an elastic material and capable of absorbing liquid in contact with the absorber;
   a support that supports the absorber; and
   a tank that receives the liquid discharged from the absorber when the absorber is compressed.
A101 The liquid collector according to Embodiment A001,
   wherein the support includes a tube that extends in a longitudinal direction.
A102 The liquid collector according to Embodiment A101,
   wherein the support includes a fixing portion that fixes the absorber in the tube.
A103 The liquid collector according to Embodiment A101 or A102,
   wherein the support includes a stopper that positions the absorber with respect to the support, the stopper having a flow hole that provides fluid communication between a side adjacent to the absorber and a side opposite to the side adjacent to the absorber in the longitudinal direction.
A104 The liquid collector according to any one of Embodiments A101 to A103,
   wherein the support has an opening at an end opposite to an end at which the absorber is fixed in the longitudinal direction, and includes a closing body that is removably attachable to the opening or capable of covering and uncovering the opening.
A111 The liquid collector according to any one of Embodiments A001 to A104,
   wherein the absorber is disposed at or near an end of the support.
A112 The liquid collector according to Embodiment A111,
   wherein the support has a shaft shape or a tubular shape that extends in the longitudinal direction, and
   wherein the absorber is fixed to one end of the support in the longitudinal direction such that the absorber is expandable.
A121 The liquid collector according to Embodiment A111 or A112,
   wherein an inside of a tube of the support serves as the tank.
A122 The liquid collector according to Embodiment A121, further including a compressing member that is movable back and forth relative to the absorber in the longitudinal direction and that compresses and deforms the absorber so that the liquid is discharged from the absorber.
A123 The liquid collector according to Embodiment A122,
   wherein the compressing member includes
      a pressing plate that is disposed to face the absorber and that presses the absorber, and
      an outer wall having a tubular shape that extends in a direction crossing the pressing plate, and
   wherein the support is insertable into a space inside the outer wall with the absorber facing the pressing plate.
A131 The liquid collector according to Embodiment A111 or A112, further including:
   a pressing body that is disposed to face the absorber and that presses the absorber,
   wherein the tank is disposed on a side of the pressing body opposite to a side on which the absorber is disposed, and
   wherein the pressing body has a flow hole that provides communication between the side on which the absorber is disposed and an inside of the tank on the side opposite to the side on which the absorber is disposed.
A141 The liquid collector according to any one of Embodiments A111 to A131,
   wherein the stopper includes a projection or a three-dimensional structure that projects toward the absorber inserted into the space inside the outer wall.
A151 The liquid collector according to any one of Embodiments A001 to A104,
   wherein the absorber is at least partially fixed to the support such that the absorber is expandable in the support when the absorber absorbs the liquid.
A152 The liquid collector according to Embodiment A151,
   wherein the support includes a tube that extends in the longitudinal direction, and
   wherein the absorber is fixed to one end of the tube in the longitudinal direction in an expandable manner in the tube, and is expandable in the longitudinal direction in the tube when the absorber absorbs the liquid.
A161 The liquid collector according to Embodiment A151 or A152,
   wherein an inside of the support serves as the tank, and
   wherein the liquid absorbed in the absorber is discharged into the tank due to compression of air from an end of the support at which the absorber is disposed.
A162 The liquid collector according to Embodiment A151 or A152,
   wherein an inside of the support serves as the tank,
   wherein the liquid collector further includes a piston that is movable back and forth relative to the absorber in the support, and
   wherein the piston includes a compressing member that compresses and deforms the absorber so that the liquid is discharged from the absorber into the tank.
A163 The liquid collector according to Embodiment A162,
   wherein the piston has a communication hole that provides communication between a side adjacent to the absorber and a side opposite to the side adjacent to the absorber.
A171 The liquid collector according to Embodiment A152,
   wherein the tank is attachable to an end of the support at which the absorber is disposed, and is capable of receiving the liquid discharged from the absorber when the absorber is compressed.
A172 The liquid collector according to Embodiment A171, further including:
   a piston that is movable back and forth in the longitudinal direction in the tube of the support,
   wherein the piston compresses and deforms the absorber in the tube so that the liquid is discharged from the absorber.
A181 The liquid collector according to any one of Embodiments A001 to A172,
   wherein a plurality of the absorbers are stacked together, or the absorber includes a plurality of absorbers that are stacked together.
A182 The liquid collector according to Embodiment A181,
   wherein the plurality of absorbers differ in at least one of a group of characteristics including color, shape, and material.
A183 The liquid collector according to Embodiment A181 or A182,
   wherein the plurality of absorbers are connected to each other.
A184 The liquid collector according to any one of Embodiments A181 to A183,
   wherein the plurality of absorbers are connected to the support by a line-shaped connecting member.
A191 The liquid collector according to any one of Embodiments A001 to A184,
   wherein the tank includes a scale mark that indicates an amount of liquid received in the tank.
A201 A liquid collector including:
   an absorber including an elastic material and capable of absorbing liquid in contact with the absorber;
   a support that supports the absorber;
   a first cylinder that supports the absorber at an end of the first cylinder and in which a liquid-receiving space that receives the liquid is defined; and
   a second cylinder into which the first cylinder is insertable and that is configured to press the absorber so that the liquid absorbed in the absorber moves into the liquid-receiving space when the first cylinder is inserted into the second cylinder.
A202 A liquid collector including:
   an absorber including an elastic material and capable of absorbing liquid in contact with the absorber;
   a first cylinder that supports the absorber at an end of the first cylinder; and
   a second cylinder into which the first cylinder is insertable and in which a liquid-receiving space that receives the liquid is defined; and
   wherein the second cylinder is configured to compress and deform the absorber so that the liquid absorbed in the absorber moves into the liquid-receiving space when the first cylinder is inserted into the second cylinder.
B101 A method for collecting liquid, including:
   causing an absorber to absorb liquid by bringing the absorber into contact with the liquid;
   causing the absorber to discharge the liquid absorbed in the absorber; and
   receiving the liquid discharged from the absorber in a tank.
B102 The method according to Embodiment B101,
   wherein the causing the absorber to discharge the liquid absorbed in the absorber includes compressing the absorber.
B103 The method according to Embodiment B101 or B102, further including:
   providing the liquid collector according to any one of Embodiments A001 to A202.
B104 The method according to any one of Embodiments B101 to B103, further including:
   estimating an amount of absorption of the liquid based on a change in a characteristic of the absorber.
B105 The method according to Embodiment B104,
   wherein the characteristic of the absorber includes one of volume and color of the absorber.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

### Reference Signs List

- 100: liquid collector
- 110: collector body
- 120: support (first cylinder)
- 121: tube
- 123: stopper
- 124: flow hole
- 127: closing body
- 130: absorber
- 140: tank
- 141: liquid-receiving space
- 150: compressing member (second cylinder)
- 151: outer wall
- 153: pressing plate
- 154: opening
- 160: air hole
- 180: connecting member
- 200: liquid collector
- 210: collector body
- 220: support (first cylinder)
- 221: tube
- 223: stopper
- 230: absorber
- 240: tank
- 241: liquid-receiving space
- 250: compressing member (second cylinder)
- 251: outer wall
- 252: bottom plate
- 253: pressing body
- 254: opening
- 255: large-diameter portion
- 257: communication hole
- 261: surface
- 300: liquid collector
- 310: collector body
- 320: support (first cylinder)
- 321: tube
- 323: stopper
- 324: flow hole
- 327: closing body
- 330: absorber
- 340: tank (second cylinder)
- 341: tubular wall
- 342: bottom plate
- 344: opening
- 345: large-diameter portion
- 346: liquid-receiving space
- 350: compressing member
- 351: piston
- 352: support shaft
- 400: liquid collector
- 410: collector body
- 420: support
- 421: tube
- 423: stopper
- 424: flow hole
- 430: absorber
- 440: tank (second cylinder)
- 441: tank body
- 442: piston
- 444: liquid-receiving space
- 445: communication hole
- 446: cap
- 447: air hole
- 450: outer cover
- 451: closing body
- 452: tubular wall
- 500: liquid
- 520: support
- 523: stopper
- 524: flow hole
- 530: absorber
- 540: tank
- 600: support surface
- 700: air
- 1211: tube end
- 1222: recess
- 1223: opening
- 2551: step portion
- 3223: opening
- 4223: opening
- Da: longitudinal direction
- S10: liquid collection method
- S11: step
- S12: step
- S13: step
- S20: liquid collection method
- S21: step
- S22: step
- S23: step
- S30: liquid collection method
- S31: step
- S32: step

## Claims

1. A liquid collector comprising:
an absorber including an elastic material and capable of absorbing liquid in contact with the absorber;
a support that supports the absorber; and
a tank that receives the liquid discharged from the absorber when the absorber is compressed.

2. The liquid collector according to Claim 1,
wherein the support includes a tube that extends in a longitudinal direction.

3. The liquid collector according to Claim 2,
wherein the support includes a fixing portion that fixes the absorber in the tube.

4. The liquid collector according to Claim 2 or 3,
wherein the support includes a stopper that positions the absorber with respect to the support, the stopper having a flow hole that provides fluid communication between a side adjacent to the absorber and a side opposite to the side adjacent to the absorber in the longitudinal direction.

5. The liquid collector according to any one of Claims 2 to 4,
wherein the support has an opening at an end opposite to an end at which the absorber is fixed in the longitudinal direction, and includes a closing body that is removably attachable to the opening or capable of covering and uncovering the opening.

6. The liquid collector according to any one of Claims 1 to 5,
wherein the absorber is disposed at or near an end of the support.

7. The liquid collector according to Claim 6,
wherein the support has a shaft shape or a tubular shape that extends in the longitudinal direction, and
wherein the absorber is fixed to one end of the support in the longitudinal direction such that the absorber is expandable.

8. The liquid collector according to Claim 6 or 7,
wherein an inside of a tube of the support serves as the tank.

9. The liquid collector according to Claim 8, further comprising:
a compressing member that is movable back and forth relative to the absorber in the longitudinal direction and that compresses and deforms the absorber so that the liquid is discharged from the absorber.

10. The liquid collector according to Claim 9,
wherein the compressing member includes
a pressing plate that is disposed to face the absorber and that presses the absorber, and
an outer wall having a tubular shape that extends in a direction crossing the pressing plate, and
wherein the support is insertable into a space inside the outer wall with the absorber facing the pressing plate.

11. The liquid collector according to Claim 8 or 9, further comprising:
a pressing body that is disposed to face the absorber and that presses the absorber,
wherein the tank is disposed on a side of the pressing body opposite to a side on which the absorber is disposed, and
wherein the pressing body has a flow hole that provides communication between the side on which the absorber is disposed and an inside of the tank on the side opposite to the side on which the absorber is disposed.

12. The liquid collector according to any one of Claims 1 to 5,
wherein the absorber is at least partially fixed to the support such that the absorber is expandable in the support when the absorber absorbs the liquid.

13. The liquid collector according to Claim 12,
wherein the support includes a tube that extends in the longitudinal direction, and
wherein the absorber is fixed to one end of the tube in the longitudinal direction in an expandable manner in the tube, and is expandable in the longitudinal direction in the tube when the absorber absorbs the liquid.

14. The liquid collector according to Claim 12 or 13,
wherein an inside of the support serves as the tank, and
wherein the liquid absorbed in the absorber is discharged into the tank due to compression of air from an end of the support at which the absorber is disposed.

15. The liquid collector according to Claim 12 or 13,
wherein an inside of the support serves as the tank,
wherein the liquid collector further comprises a piston that is movable back and forth relative to the absorber in the support, and
wherein the piston includes a compressing member that compresses and deforms the absorber so that the liquid is discharged from the absorber into the tank.

16. The liquid collector according to Claim 15,
wherein the piston has a communication hole that provides communication between a side adjacent to the absorber and a side opposite to the side adjacent to the absorber.

17. The liquid collector according to Claim 13,
wherein the tank is attachable to an end of the support at which the absorber is disposed, and is capable of receiving the liquid discharged from the absorber when the absorber is compressed.

18. The liquid collector according to Claim 17, further comprising:
a piston that is movable back and forth in the longitudinal direction in the tube of the support,
wherein the piston compresses and deforms the absorber in the tube so that the liquid is discharged from the absorber.

19. The liquid collector according to any one of Claims 1 to 18,
wherein the absorber comprises a plurality of absorbers that are stacked together.

20. The liquid collector according to Claim 19,
wherein the plurality of absorbers differ in at least one of color, shape, and material.

21. The liquid collector according to Claim 19 or 20,
wherein the plurality of absorbers are connected to each other.

22. The liquid collector according to any one of Claims 19 to 20,
wherein the plurality of absorbers are connected to the support by a line-shaped connecting member.

23. The liquid collector according to any one of Claims 1 to 22,
wherein the tank includes a scale mark that indicates an amount of liquid received in the tank.

24. A liquid collector comprising:
an absorber including an elastic material and capable of absorbing liquid in contact with the absorber;
a support that supports the absorber;
a first cylinder that supports the absorber at an end of the first cylinder and in which a liquid-receiving space that receives the liquid is defined; and
a second cylinder into which the first cylinder is insertable and that is configured to press the absorber so that the liquid absorbed in the absorber moves into the liquid-receiving space when the first cylinder is inserted into the second cylinder.

25. A liquid collector comprising:
an absorber including an elastic material and capable of absorbing liquid in contact with the absorber;
a first cylinder that supports the absorber at an end of the first cylinder; and
a second cylinder into which the first cylinder is insertable and in which a liquid-receiving space that receives the liquid is defined,
wherein the second cylinder is configured to compress and deform the absorber so that the liquid absorbed in the absorber moves into the liquid-receiving space when the first cylinder is inserted into the second cylinder.

26. A method for collecting liquid, comprising:
causing an absorber to absorb liquid by bringing the absorber into contact with the liquid;
causing the absorber to discharge the liquid absorbed in the absorber; and
receiving the liquid discharged from the absorber in the tank.

27. The method for collecting liquid according to Claim 26,
wherein the causing the absorber to discharge the liquid absorbed in the absorber comprises compressing the absorber.

28. The method for collecting liquid according to Claim 26 or 27, further comprising:
providing the liquid collector according to any one of Claims 1 to 25.

29. The method according to any one of Claims 26 to 28, further comprising:
estimating an amount of absorption of the liquid based on a change in a characteristic of the absorber.

30. The method according to Claim 29,
wherein the characteristic of the absorber includes one of volume and color of the absorber.
